Europäisches Patentamt

European Patent Office

Office européen des brevets

(19)

(11) Publication number: **0 030 812**
**B1**

(12) **EUROPEAN PATENT SPECIFICATION**

(45) Date of publication of patent specification: **14.03.84**

(51) Int. Cl.³: **A 61 K 35/78**

(21) Application number: **80304337.1**

(22) Date of filing: **02.12.80**

(54) A process for preparing substances having interferon inducing activity and interferon inducers.

(30) Priority: **03.12.79 JP 156627/79**
**27.12.79 JP 170642/79**

(43) Date of publication of application:
**24.06.81 Bulletin 81/25**

(45) Publication of the grant of the patent:
**14.03.84 Bulletin 84/11**

(84) Designated Contracting States:
**DE FR GB IT**

(56) References cited:
**FR - A - 2 439 586**
**GB - A - 2 036 751**
**GB - A - 2 042 555**
**GB - A - 2 042 558**
**NL - C - 63 333**
**US - A - 2 242 062**
**US - A - 3 219 542**

**CHEMICAL ABSTRACTS, vol. 69, March 16, 1964, No. 6, ref. 6701a Columbus, Ohio, US E. STEINEGGER et al.: "Analgesic principle of the roots of Ecballium elaterium"**

(73) Proprietor: **KITASATO KENKYUSHO**
**9-1, Shirokane 5 chome Minato-ku**
**Tokyo-to (JP)**

(72) Inventor: **Kojima, Yasuhiko**
**244-9, Kosugaya-cho Totsuka-ku**
**Yokohama-shi Kanagawa-ken (JP)**
Inventor: **Tamamura, Sadao**
**1-40-5, Shimouma Setagaya-ku**
**Tokyo-to (JP)**
Inventor: **Konno, Seishi**
**415-1-1203, Kuwakawa-cho Edogawa-ku**
**Tokyo-to (JP)**
Inventor: **Hashimoto, Takashi**
**1-21, Kami-ishihara Chofu-shi**
**Tokyo-to (JP)**

(74) Representative: **Watkins, Arnold Jack et al,**
**European Patent Attorney Frank B. Dehn & Co.**
**Imperial House 15-19 Kingsway**
**London WC2B 6UZ (GB)**

Courier Press, Leamington Spa, England.

(56) References cited:

**CHEMICAL ABSTRACTS, vol. 65, October 24, 1966, no. 9 ref. 14300g Columbus, Ohio, US M.M. LOTLIKAR et al.: "Pharmacology of a hypoglycemic principle isolated from the fruits of Momordica charantia"**

**CHEMICAL ABSTRACTS, vol. 58, April 29, 1963, no. 9, ref. 9537d Columbus, Ohio, US M.M. LOTLIKAR et al.: "Note on a hypoglycemic principle isolated from the fruits of Momordica charantia"**

A process for preparing substances having interferon inducing activity and interferon inducers

This invention relates to a process for preparing substances having interferon inducing activity and to interferon inducers *per se*. More particularly the invention relates to the extraction of interferon inducers from the tissue of higher plants.

It is known that substances having interferon inducing activity have been extracted from certain plants. Thus a substance having interferon inducing activity has been extracted, using hot water, from the root of *Angelica acutiloba* Kitagawa (known in Japan as *Toki*). This extraction is described in Japanese Patent Application laid open to public inspection as Kokai Koho 32107/78. Interferon inducing substances have also been isolated from mulberries such as *Morus alba* L. or *Morus bombycis* Koidz., as disclosed in a Japanese Patent Application laid open to public inspection as Kokai Koho 99313/78. Both *Morus alba* L. and *Morus bombycis* Koidz. have been used as traditional Sino-Japanese medical herbs. Such interferon inducers have been prepared using hot water to give an extracted solution which may, if desired, be reduced to a suitable quantity by concentration under reduce pressure or by the use of a Diaflo membrane, adding an organic solvent such as e.g. acetone to the extracted solution to give a precipitate containing the said active substance and subjecting the precipitate to dialysis, followed by freeze-drying.

It has not, hitherto, been known that plants of the gourd family (*Cucurbitaceae*) contain a substance having interferon inducing activity and of course no process for the isolation of such a substance therefrom has been described to the applicants knowledge. U.S. Patents Nos. 2,242,062 and 3,219,542, Dutch Patent No. 63333 and Chemical Abstracts Vol 68 (1963): 9537d each describe the extraction of substances from plants of the gourd family (*Cucurbitaceae*), but there is no teaching or even suggestion that the substances isolated are IF inducers and the properties of the isolated substances are not consistent with the properties of an IF inducer. Moreover the process steps described result in the isolation of substances other than the IF inducers obtained according to the present invention for example as a result of effecting the extraction or isolation using organic solvents in which the IF inducer of the present invention is insoluble or effecting the extraction or isolation under conditions which result in the destruction of the IF inducer of the present invention.

The term interferon inducer used herein denotes a substance capable of acting upon human and animal cells to induce the production of interferon (also designated as IF). Thus, an IF inducer is of potential interest because of its use in various biochemical studies and in the cure and treatment of various human and animal diseases caused by viral infection.

The present invention is based upon the discovery that substances isolated from the tissues, e.g. seeds, of various plants of the gourd family (*Cucurbitaceae*) exhibit high IF inducing activity. Moreover such IF inducers may readily be isolated from the plant tissues.

According to one feature of the present invention, there is provided a process for preparing a water-soluble IF inducer which comprises extracting the said IF inducer with water or phenol/water from the tissue of a plant of the gourd family (*Cucurbitaceae*) or a variant thereof containing the said IF inducer whereby to form a supernatant and isolating the said IF inducer from the supernatant thereby obtained.

Various plants belonging to the gourd family are grown in the wild and/or are cultured in various countries of the world. It is said, for example, that in Japan, 5 genera and 10 species are grown in the wild and 8 genera and 10 species are cultured for use as foodstuffs or folk cures. These plants include, for example, *Cucumis melo* L., *Lagenaria siceraria* L. and the like. Although any and all plants of *Cucurbitaceae* may be used for the purpose of this invention when they contain an IF inducer it is convenient to use plants selected from the genera *Benincasa, Cucurbita, Citrullus, Cucumis, Lagenaria, Luffa, Momordica, Tricosanthes* and variants thereof. It is preferred to use plants selected from the following:

| Botanical name | Common name |
|---|---|
| *Benicasa hispida* Cogn.; syn. *B. cerifera* Saal | Tougo*, Tougan*, Kamouri* (Wax gourd) |
| *Cucurbita moschata* Dec. | Nihon-kabocha*, Tounasu* (pumpkin) |
| *Cucurbita pepo* L. | somen-kabocha*, Ito-kabocha*, Kinshi-uri* |
| *Cucurbita maxima* Dec | Kuri-kabocha* |
| *Citrullus vulgaris Schr.* | Water melon |
| *Cumumis melo* L. | Melon |
| *Cucumis sativus* L. | Cucumber |
| *Lagenaria siceraria* Stand. | Yugao*, Fukuba*, Hyotan* (Gourd) |
| *Luffa cylindrica* Roem. | Sponge gourd |
| *Momordica charantis* L. | Niga-uri*, Tsuru-reishi* |
| *Tricosanthes cucumeroides* Maxim. | Karasu-uri* |
| *Tricosanthes kirilowii* Maxim. var *japonica* Kitam. | Kikarasu-uri* |

(*indicates the name of the plant commonly used in Japan)

Although IF inducers may be extracted from all tissues of the plant, e.g. the stem, root and leaf we have found that the seeds are particularly rich in the IF inducers of the present invention.

The following discussion is thus made with specific reference to the use of seeds, but it will be appreciated that the extraction of IF-inducing substance(s) from plant tissue other than the seed(s) falls within the scope of the present invention. It is preferred to use the dried seed for better preservation and extraction, although the fresh seed may be used if desired. The seed consists of hard hull, thin internal seed coat and cotyledon, and the IF-inducing substance is mainly present in the hull and cotyledon. In order to effect the extraction with better efficiency, it is preferred to break the hull prior to extraction.

When oils and fats, sterols, pigments and various other impurities contained in the cotyledon are extracted with a suitable organic solvent such as e.g. methanol, ethanol, chloroform, ether, hexane or a mixture thereof prior to extraction with water, the purity and yield of the extracted solution may be improved without loss of the active substance. If desired, it is also possible to extract the hull and its ingredients separately.

Extraction with water may, in general, be effected by using hot water (for example, 5—10 times by weight of the seeds) for example at 50—120°C, preferably 60—100°C for 30 minutes to 2 hours. Better efficiency may be obtained by adjusting the pH of the extracting water for example to 7—10 with an alkali such as e.g. sodium hydroxide, potassium hydroxide, ammonium hydroxide and the like. The extracted solution thus obtained may contain pigments, low molecular weight substances and various other impurities besides the active substance of this invention, and these impurities may be removed by ultrafiltration.

In a further embodiment, it is possible to treat with phenol the solution obtained by extraction of the seed with water. In this case, the extracted solution may, if desired, be concentrated to a suitable amount. After this, the extracted solution is treated with phenol to give a concentration of phenol, for example, of 40—55% in the mixed solution. By heating the mixed solution for example to 60—100°C, water and phenol are well-mixed to give a uniform liquid phase. The uniform solution is conveniently kept at this temperature for example for 20 minutes to 2 hours and then for example rapidly cooled to a suitable temperature (for example, room temperature) so that the water separates from the phenol to give a whitish turbid solution. This solution is allowed to stand, for example at room temperature or centrifuged (e.g. at 10—20°C for 10—30°C minutes at 3000—10,000 r.p.m.) so that the solution separates into the water layer and phenol layer. The water layer is rich in the active substance. After removal of the water layer, the lower layer i.e. phenol layer is conveniently treated with water in a substantially equal amount to the amount of the removed water. By repeating the above-mentioned heating, rapid cooling and separating treatments, it is possible to recover a major portion of the active substance contained in the extracting solution (in some cases, more than 90%). The thus-obtained water layers are collected and combined, and the combined water layers are treated in conventional manner (e.g. dialysis or ether extraction) to remove the phenol content. Although it is possible if desired to effect extraction with a phenol/water mixture as a first stage, it is preferred to use water in the absence of phenol as a first stage for ease of operation and economy. However, when compared with treatment using water alone, the phenol treatment may serve to improve the purity of the final product, although the operation may be complicated and expensive. Although the reason for such improvement has not yet been clarified, it is believed that partial purification may be effected by phenol treatment so that impurities such as proteins may be removed. The purity of the final product obtained by extraction with water is better than the purity of the corresponding final product obtained by phenol treatment, when the crude products are purified by the same procedure.

4

# 0 030 812

Instead of the above-mentioned phenol treatment or in combination with it, it may be possible to form a precipitate containing the active substance, for example (a) by addition of a hydrophilic organic solvent to the extracted solution which may, if desired, be concentrated prior to the addition of the organic solvent, (b) by fractionating the precipitate by the salting out method, or (c) by adding an alkaline reagent (e.g. Benedict reagent), quaternary ammonium ions, trypaflavine or rivanol to the extracted solution. However, these treatments are complicated and moreover no additional advantage may be obtained when compared with the extraction with water.

The solution resulting from the extraction with water or from phenol treatment may contain impurities such as e.g. pigments or low molecular weight substances, which may if desired be removed by ultrafiltration. Such a removal may be effected before or after the above-mentioned phenol treatment. However, it is necessary in such a case to remove phenol, ethyl ether and other solvents, which may injure the ultrafiltration membrane prior to ultrafiltration by any appropriate method, for example, by dialysis or concentration *in vacuo*.

It is important to select the molecular weight range to be fractionated and thus the ultrafiltration membrane to be used. This range may vary depending on the type of plant used as starting material.

The active substances obtained by the process of the present invention are water-soluble acidic polysaccharides having a high molecular weight, although all their physicochemical characteristics are not yet clear. When various purified products of this invention are independently dissolved in water, almost all solutions are able to pass an ultrafiltration membrane capable of fractionating substances having a molecular weight of 50,000 calculated as globular protein (e.g. UK-50, commercial product of Toyo Roshi K.K., Tokyo), while some solutions can pass through a membrane capable of fractionating substances having a molecular weight of 200,000 (as globular protein; e.g. UK-200, commercial product of Toyo Roshi K.K., Tokyo) and other solutions can hardly pass through the same, depending upon the type of plant used as starting material. It is thus important to select the type of membrane to be used. Examples of the former are the active substances from the seeds of sponge gourd, gourd and the like, and the latter type are exemplified by the active substances isolated from the seeds of the pumpkin, water melon etc.

Table 1 shows whether certain active substances obtained from the seeds of various plants of the gourd family are able to pass through certain membranes, in which "+" indicates that a major portion of the active substance can hardly pass through UK-50 but can pass through UK-200, and "−" indicates that the major portion of the active substance can hardly pass through UK-50 and UK-200 membranes when they are subjected to ultrafiltration effected under a normal pressure of 1—5 kg/cm$_2$.

## TABLE 1

| Botanical name (common name) | Permeability |
| --- | --- |
| *Benincasa hispida* Cogn.; syn. *B. cerifera* Saal (Wax gourd) | − |
| *Cucurbita moschata* Dec. (pumpkin, Nihon-kabocha*) | + |
| *Cucurbita pepo* L. (Kinshi-uri*) | + |
| *Cucurbita maxima* Dec. (Kuri-kabocha*, pumpkin) | + |
| *Citrullus vulgaris* Schr. (Water melon) | + |
| *Cucumis melo* L. (Melon) | − |
| *Cucumis sativus* L. (Cucumber) | − |
| *Lagenaria siceraria* Stand. (Gourd) | − |
| *Luffa cylindrica* Roem. (Sponge gourd) | − |
| *Momordica charantis* L. (Niga-uri*) | − |
| *Tricosanthes cucumeroides* Maxim. (Karasu-uri*) | − |
| *Tricosanthes kirilowii* Maxim. var *japonica* Kitam. (Kikarasu-uri*) | − |

According to a further feature of the present invention there is thus provided a polysaccharide IF inducer extracted from the tissue of a plant of the gourd family which is stable in the form of an amorphous whitish powder and which in substantially pure form possesses the following physico-chemical characteristics:

(1) Molecular weight: 30,000 to 200,000
(2) Melting point or decomposition point: Melting point indefinite. Carbonized at about 220°C;
(3) Ultraviolet absorption spectrum: Maximum absorption at about 258 nm; Colour reaction obtained by phenol/sulphuric acid method maximum absorption at about 490 nm;
(4) Solubility in various solvents: Readily soluble in water and aqueous solutions of an alkali; Soluble in saturated aqueous solutions of phenol and dimethylsulfoxide; and substantially insoluble in methanol, ethanol, propanol, butanol, acetone, chloroform; diethyl ether, benzene and hexane;
(5) Reactions: Positive in Molish reaction, chromotropic acid reaction, tryptophan reaction and orcinol reaction, and negative in diphenylamine reaction;
(6) Nature: Acidic;

5

(7) Main chemical constituents: Phosphoric acid; rhammose, arabinose, galactose and glucose moieties; xylose and/or mannose moieties; galacturonic acid and/or glucuronic acid moieties and amino compounds.

The IF inducers are preferably in substantially pure form.

The IF inducers obtained by the process of this invention are acidic high molecular weight polysaccharides and are readily soluble in water, in particular, under alkaline conditions. Thus it is possible to purify any of the IF inducers of this invention by appropriate methods or modifications thereof. Such methods for purifying IF inducers of this type include, for example, gel filtration, ion exchange chromatography, affinity chromatography and electrophoresis. After this, various impurities such as e.g. salts may, if desired, be removed, for example, by gel filtration, reverse osmosis and various other known methods. Finally, the IF inducers may be obtained in high purity by freeze-drying.

The IF inducers isolated from the seeds of *Cucurbitaceae* are stable in the form of whitish amorphous acidic nitrogen containing polysaccharides. Their molecular weights tend to differ depending upon the type of starting plant used. When dissolved in water, the solutions are slightly viscous and contain phosphoric acid. The IF inducers are readily soluble in water, in particular, under alkaline conditions and are also soluble in saturated aqueous solutions of phenol and dimethylsulfoxide, but insoluble in methanol, ethanol, propanol, butanol, ether, acetone, chloroform, benzene and hexane. Decomposition of the active substances with dilute sulfuric acid or trifluoroacetic acid, yields neutral sugars, uronic acids and amino compounds. Although the sugar compositions and their ratios may vary, depending upon the type of starting plant used, rhamnose, arabinose, galactose and glucose are neutral sugars common to all IF inducers of the present invention. Moreover, at least one of xylose and mannose is found in all IF inducers of the present invention, depending upon the type of starting plants used.

Uronic acids present in IF inducers of the present invention include galacturonic acid which is replaced by glucuronic acid in some cases. The presence of amino sugars is not observed. Various ultra violet absorption spectra obtained by using different plants show generally a maximum absorption at about 258 nm, and the colour reaction obtained by the phenol/sulfuric acid method exhibit a maximum absorption at about 490 nm. The IF inducers of the invention are positive in the Molish reaction, chromotropic acid reaction, tryptophan reaction and orcinol reaction, and negative in the diphenylamine reaction. Although their melting or decomposition points are indefinite, the IF inducers are carbonized at about 220°C.

As stated above it is also possible to extract IF inducers from tissue other than the seeds, i.e. the stem, leaf and/or root of various plants of *Cucurbitaceae* containing the said IF inducer and recover the same from the extract thereby obtained in a similar manner to that described above.

Either fresh or dried stems, leaves and/or roots of various plants of *Cucurbitaceae* may be used for this purpose. Although all the physical and chemical characteristics of the IF inducers present in the stems and/or leaves are not yet clear, they have lower heat stability than that of the active substances contained in the seeds so that their interferon-inducing activities are substantially inactivated when the extraction is effected, for example, with water at 100°C for 2 hours. Also, their interferon-inducing activities are considerably lowered by extraction with a phenol/water mixture. Thus, it is preferred to effect the extraction with water at a pH of 7—10 for example for 20 minutes to 2 hours at a temperature from ambient to 70°C.

The roots of the plants of *Cucurbitaceae* (for example, the roots of the genus *Tricosanthes*) are long and massive and contain a large amount of starch and thus it is preferred to effect the extraction with water at a temperature which is lower than the gelatinizing temperature of the starch (e.g. not higher than 60°C), although it is possible to effect the extraction with a phenol/water mixture at such a relatively low temperature. The physico-chemical characteristics of the active substances obtained from the roots are not yet clear, although it is possible to purify the extracted solutions in a similar manner to that described above. It has been found that the highest activity of the active substances extracted from the roots is capable of passing through an ultrafiltration membrane capable of fractionating substances having a molecular weight, for example, of 30,000 and which is incapable of passing through a cellophane tube used for dialysis. Thus, it is important to select a suitable ultrafiltration membrane, depending upon the types of the starting materials. The extraction time may, for example, be equal to that required for the water extraction from the roots. The activity of the active substances obtained from the roots is in general equal to or lower than that of the substances obtained from the seeds. The physico-chemical characteristics of the active substances obtained from the roots are not yet clear. However, the extraction and purification may be effected in a similar manner to that used for extraction from the seeds.

The following non-limiting Examples illustrate the invention. Dried plants are used as starting materials, unless otherwise specified. The IF inducing activities of the products obtained by the process of this invention were determined by the methods hereinafter described in the experiments. The plant names marked "*" are the common names employed in Japanese usage.

Example 1

Touga* (*Benincasa hispida* Cogn. synonym of *B. cerifera* Saal:

Dried seeds (400 g) of the plant were coarsely crushed and dipped in a mixture of chloroform/methanol (2:1; 1,000 ml) for 2 days at room temperature. The dipping was effected twice to remove oils and fats from the seeds and the solvent was completely removed by evaporation. Water (3,000 ml) was added to the sample (300 g) which was then heated for one hour in a water bath kept at 90—100°C. The sample was filtered to give a filtrate. The addition of water (3,000 ml) to the sample, heating, extraction and filtration were repeated three times, and all filtrates were combined. The combined filtrates (8,000 ml) were concentrated under reduced pressure to an amount of 1,100 ml, to which an equal amount of 90% phenol was added, and the combined solutions were kept in a water bath (70 to 80°C) for one hour with occasional agitation. The solution thus obtained possessed a uniform yellowish brown colour and was clear. By rapid cooling in a stream of water, a whitish turbid solution was obtained, which was then centrifuged (4,000 r.p.m.) at 10°C for 20 minutes to separate the water layer (800 ml). Water (800 ml) was added to the lower layer and the mixture subjected to heating four times, cooling and separation taking place in a similar manner to that described above to give four water layers. Each water layer was treated with diethyl ether (200 ml) and the phenol was removed by extraction with shaking. The ether extraction was effected three times in this manner. All water layers were collected and combined and ether was removed by evaporation. The combined solutions were then subjected to ultrafiltration using an ultra-filter (UHP-76, commercially available from Toyo Roshi K.K., Tokyo) with UK-200 membrane (commercially available from Toyo Roshi K.K.) capable of fractionating substances having a molecular weight of 200,000 to give a residue which was then freeze-dried to obtain a crude product (730 mg). This crude product (300 mg) was dissolved in a tris-HCl buffer solution (pH 8.0; I=0.1; 20 ml) and transferred to a column (26×1,000 mm) packed with Sephacryl S-300 (commercially available from Pharmacia Fine Chemicals AB., Sweden). A similar buffer solution was used for elution. The eluant was divided into fractions (each 5 ml). In accordance with the absorption at 258 nm and with the sugar determined by the phenol/sulfuric acid reaction, the fractions corresponding to the first peak were collected and combined, and the combined fractions were desalted by using an ultrafiltration membrane (UH-50; MW=5,000; commercial product of Toyo Roshi K.K., Tokyo) to give a residue which was then freeze-dried to obtain a final product (100 mg) in the form of the white amorphous solid.

Example 2

Nihon-kabocha* (*Cucurbita moschata* Dec.)

The hull (110 g) was treated in a similar manner to that described in Example 1 except that the fractions capable of passing through a membrane UK-200 (capable of fractionating substances having a molecular weight of 200,000) and incapable of passing through a membrane UK-50 (capable of fractionating substances having a molecular weight of 50,000) were collected and combined, and the combined fractions were freeze-dried to give a crude product (530 mg), 100 mg of which was transferred to a column (26×400 mm) packed with ECTEOLA cellulose (commercial product of Serva Entwicklungslabor, German Federal Republic) treated with a similar tris-HCL buffer solution to that used in Example 1. The elution was also effected in a similar manner to that described in Example 1. The absorption at 258 nm and the determination of sugars were effected in a similar manner to that described in Example 1. After no sugar was observed, a further elution was effected by using a similar buffer solution together with 0.3 M sodium chloride (200 ml). The eluant was treated in a similar manner to that described in Example 1 to obtain a final product in the form of a white amorphous solid (20 mg).

Example 3

Nihon-kabocha* (*Cucurbita moschata* Dec.)

The extraction was effected with hot water using the cotyledon (95 g) of the title plant in a similar manner to that described in Example 1, the filtrate being treated with phenol and 500 mls of water being used on each occasion. The solutions obtained were then subjected to ultrafiltration in a similar manner to that described in Example 2 to obtain a crude product (85 mg) which was transferred to a column (25×400 mm) packed with Bio-Gel P-200 (commercial product of Bio-Rad Laboratories Ltd., U.S.A.) and treated in a similar manner to that described in Example 1 to yield a white amorphous solid (15 mg).

Example 4

Somen-kabocha* (*Cucurbita pepo* L.)

The seeds (30 g) were treated in a similar manner to that described in Example 1 to obtain a crude product (95 mg), 90 mg of which were treated in a similar manner to that described in Example 3 to obtain a white amorphous solid (18 mg).

Example 5

Kuri-kabocha* (*Cucurbita maxima* Dec.)

The seeds (200 g) were crushed coarsely and defatted, extracted and treated with phenol in a

**O 030 812**

similar manner to that described in Example 1 and subjected to ultrafiltration in a similar manner to that described in Example 2 to obtain a crude product (615 mg), 300 mg of which were transferred to a column (26×1000 mm) packed with DEAE Sephadex A-25 (commercially available from Pharmacia Fine Chemicals AB., Sweden) and treated in a similar manner to that described in Example 2 to yield a white amorphous solid (43 mg).

Example 6
Suika* (*Citrullus volgaris* Schr.)
The seeds (30 g) were treated in a similar manner to that described in Example 1 to obtain a crude product (45 mg) which was transferred to a column (16×400 mm) packed with DEAE Sephadex A-25 and treated in a similar manner to that described in Example 2 to yield a white amorphous solid (10 mg).

Example 7
Melon (*Cucumis melo* L.)
The fresh seeds (60 g) of Honey Dew melon from U.S.A. were treated in a similar manner to that described in Example 1 to obtain a crude product (103 mg), of which 100 mg were treated in a similar manner to that described in Example 3 to yield a white amorphous solid (15 mg).

Example 8
Ki-uri* (*Cucumis sativus* L.)
The seeds (50 g) of Shimotsuki Aonaga Ki-uri* were treated in a similar manner to that described in Example 1 to obtain a crude product (140 mg). The crude product was treated in a similar manner to that described in Example 2 except that DEAE Sephadex A-25 was used instead of ECTEOLA cellulose to yield an amorphous whitish solid (30 mg).

Example 9
Yugao* (*Lagenaria siceraria* Stand.)
The seeds of Sakigake Yugao* (100 g) were treated in a similar manner to that described in Example 1 to obtain a crude product (125 mg), 100 mg of which were treated in a similar manner to that described in Example 1 to yield an amorphous whitish solid (35 mg).

Example 10
Hechima* (*Luffa cylindrica* Roem.)
The seeds of Onaga Hechima* (100 g) were treated in a similar manner to that described in Example 1 to obtain a crude product (110 mg), 100 mg of which were treated in a similar manner to that described in Example 1 to yield an amorphous whitish solid (25 mg).

Example 11
Niga-uri* (*Momordia charantis* L.)
The seeds of Onaga Nishaku Niga-uri* (40 g) were treated in a similar manner to that described in Example 1 to obtain a crude product (147 mg), 100 mg of which were treated in a similar manner to that described in Example 1 to give an amorphous whitish solid (22 mg).

Example 12
Karasu-uri* (*Tricosanthes cucumeroides* Maxim.)
The hulls (100 g) were treated in a similar manner to that described in Example 1 to obtain a crude product (200 mg), 100 mg of which were treated in a similar manner to that described in Example 1 to yield an amorphous whitish solid (35 mg).

Example 13
Kikarasu-uri* (*Tricosanthes kirilowii* Maxim)
In a similar manner to that described in Example 1 the hulls were defatted and the treated hulls (56 g) were processed to obtain a crude product (150 mg), 140 mg of which was transferred to a column (26×400 mm) packed with Sephadex G-200 (commercial product of Pharmacia Fine Chemicals AB., Sweden). Subsequent processing was conducted in a similar manner to that described in Example 1 to yield an amorphous whitish solid (25 mg).

Example 14
Kikarasu-uri* (*Tricosanthes kirilowii* Maxim. *var. japonica Kitam.*)
In a similar manner to that described in Example 1 the cotyledon was defatted and the thus-obtained sample (36 g) processed to obtain a crude product (120 mg), 100 mg of which was treated in a similar manner to that described in Example 3 to yield an amorphous whitish solid (20 mg).

Example 15
The stem of Cucumis sativus L. (cucumber) (40 g) was cut into small pieces and dipped in water

8

(600 ml) at 65°C for 2 hours. The extracted solution was filtered to remove solids which were then washed with water (200 ml). The filtrate was combined with the washing liquid, and the combined solution (550 ml) was subjected to ultrafiltration using a membrane capable of fractionating substances having a molecular weight of 30,000 (YM-30, commercial product of Amicon Corpn. U.S.A.) at a pressure of 2 kg/cm². The fractions passing through the membrane were removed and the residue freeze-dried to give a crude product (480 mg), 400 mg of which was treated in a similar manner to that described in Example 1 to yield an off-white solid (52 mg).

Example 16

The root of *Tricosanthes kirilowii* Maxim. var. japonica Kitam. was crushed and 100 g of the crushed root was dipped in water (400 ml) over night. After this, 90% phenol (400 ml) was added to the solution which was heated at 70—80°C for one hour. After cooling, the white turbid liquid phase was collected and centrifuged to obtain a clear liquid phase (100 ml). The remaining phenol phase was treated with water (100 ml) and the mixture added to the residue resulting from the filtration. In this manner, the heating, cooling and separation were repeated four times, resulting in a water phase of 565 ml in total. This phase was subjected to dialysis using a cellophane tube and then subjected to ultrafiltration using a membrane capable of fractionating substances having a molecular weight of 50,000 (UK-50, commercial product of Toyo Roshi K.K., Tokyo) to give a filtrate which was concentrated *in vacuo* and freeze-dried to yield an off-white solid (75 mg).

Experiment 1
IF induction with IF inducer and IF assay: [Reference: Y. Kojima's report in Kitasato Arch. Exp. Med., *43*:35 (1970)]
(a) IF induction *in vitro*:

A rabbit (weight about 1 kg; New Zealand White: SPF) was killed by cardiac puncture and its spleen, bone marrow and lymph node cells were collected and combined together to prepare a cell suspension containing the mixed cells ($10^7$ cells/ml) which was divided into samples (each 1 ml). To four samples, were added respectively 10, 1.0, 0.1 and 0.01 g/ml of an IF inducer (prepared in a similar manner to that described in Example 1) and each sample was cultured at 25°C for 24 hours, followed by centrifugation to obtain supernatants, each of which was used as a sample to determine the IF activity induced.

(b) IF induction *in vivo*:

An IF inducer obtained in a similar manner to that described in Example 1 was dissolved in water (500 µg/ml) and injected into the auricular vein of a rabbit (weight about 1 kg, New Zealand White, SPF). 1, 2, 4 and 6 hours after the administration, a 2 ml sample of blood was removed on each occasion from the test animal and the serum of each sample was isolated from the blood and used as a sample for determining the activity of the IF induced.

(c) Determination of IF activity:

In both methods (a) and (b), Vesicular stomatitis virus was used as the challenge virus in order to determine the activity of the IF induced in the following manner. A monolayer culture of the lined cells RK-13 of the rabbit was put in a dish and a predetermined amount of the sample of the solution obtained by the above-mentioned method (a) or (b) was added thereto. The culture was incubated at 37°C overnight with addition of Vesicular stomatitis virus. The IF activity was determined from the reduction ratio of plaques. The unit of the IF activity is expressed by the reciprocal number of the highest dilution of the sample required for reducing the numbers of plaques to 50%.

Tables 2 (*in vitro*) and 3 (*in vivo*) indicate the results of experiments carried out in the above-mentioned manner by using the IF inducers obtained by the process of this invention.

TABLE 2 (*in vitro*)

| Botanical name. (common name) | Concentration of sample (μg/ml) | | | |
|---|---|---|---|---|
| | 10. | 1. | 0.1 | 0.01 |
| (*—Japanese common name) | IF activity | | | |
| *Benincasa hispida* Cogn. (Touga*, Wax gourd) | >100 | >100 | 70 | 30 |
| *Cucurbita moschata* Dec. (Nihon-kabocha*, pumpkin) | >100 | >100 | >100 | 70 |
| *Cucurbita pepo* L. (Somen-kabocha*) | >100 | >100 | >100 | 50 |
| *Cucurbita maxima* Dec. (Kuri-kabocha*) | >100 | >100 | >100 | >100 |
| *Citrullus vulgaris* Schr. (Water melon, Suika*) | >100 | 90 | 40 | <10 |
| *Cucumis melo* L. (Melon) | >100 | 50 | 10 | <10 |
| *Cucumis sativus* L. (Cucumber) | 90 | 70 | 30 | <10 |
| *Lagenaria siceraria* Stand. (Gourd) | >100 | >100 | 80 | 40 |
| *Luffa cylindrica* Roem. (Sponge gourd) | >100 | 90 | 50 | <10 |
| *Momordica charantis* L. (Niga-uri*) | 80 | 30 | 10 | <10 |
| *Tricosanthes cucumeroides* Maxim. (Karasu-uri*) | >100 | 40 | 20 | <10 |
| *Tricosanthes kirilowii* Maxim. var. *japonica* Kitam. (Kikarasu-uri*) | >100 | 60 | 30 | <10 |
| *Cucumis sativas* L. (stem)** | >80 | 40 | <10 | <10 |
| *Tricosanthes kirilowii* (root) Maxim. var. *japonica* Kitam.** (root) | >100 | 50 | 20 | <10 |

[Note:— Seeds were used in each case except were marked **]

10

O 030 812

TABLE 3 (*in vivo*)

| Example no. | Botanical name (common name) (*—Japanese common name) | Mean value of the IF activity in the serum (2 hours after administration) (1 mg/rabbit) |
|---|---|---|
| 1 | *Benincasa hispida* Cogn. (Touga*, Wax gourd) | 150±25% |
| 2 & 3 | *Cucurbita moschata* Dec. (Nihon-kabocha*, pumpkin) | 200±20% |
| 4 | *Cucurbita pepo* L. (Somen-kabocha*). | 180±25% |
| 5 | *Cucurbita maxima* Dec. (Kuri-kabocha*) | 300±45% |
| 6 | *Citrullus vulgaris* Schr. (Water melon, Suika*) | 130±45% |
| 7 | *Cucumis melo* L. (Melon) | 50±15% |
| 8 | *Cucumis sativus* L. (Cucumber) | 95±25% |
| 9 | *Lagenaria siceraria* Stand. (Gourd) | 180±30% |
| 10 | *Luffa cylindrica* Roem. (Sponge gourd) | 120±20% |
| 11 | *Momordica charantis* L. (Niga-uri*) | 40±12% |
| 12 | *Tricosanthes cucumeroides* Maxim. (Karasu-uri*) | 120±20 |
| 13 & 14 | Tricosanthes kirilowii Maxim. var. *japonica* Kitam. (Kikarasu-uri*) | 140±30% |
| 15 | *Cucumis sativas* L. (stem)** | 120±25% |
| 16 | *Tricosanthes kirilowii* Maxim. var. *japonica* Kitam. (root)** | 170±30% |

[All samples use seeds except those marked **. Mean value was obtained using 2 rabbits].

Experiment 2
Identification of IF inducer

The antiviral factors prepared by methods (a) and (b) in Experiment 1 share the properties generally attributed to interferon such as sensitivity to inactivation by 0.08% of trypsin (after reaction at 37°C for 2 hours), inhibition of Vesicular stomatitis virus and Vaccinia virus in RK-13 rabbit cell cultures, and absence of antiviral activity against Vesicular stomatitis virus in L cells.

## Claims

1. A process for preparing a water-soluble IF inducer characterized in that the said IF inducer is extracted with water or phenol/water mixture from the tissue of a plant of the gourd family (*Cucurbitaceae*) or a variant thereof containing the said IF Inducer whereby to form a supernatant and isolating the said IF inducer from the supernatant thereby obtained.

2. A process as claimed in claim 1 wherein the plant is selected from the genera *Benincasa, Cucurbita, Citrullus, Cucumis, Lagenaria, Luffa, Momordica, Tricosanthes* and variants thereof.

3. A process as claimed in claim 2 wherein the plant is selected from *Benincasa hispida* Cogn.; *Cucurbita moschata* Dec.; *Cucurbita pepo* L.; *Cucurbita maxima* Dec.; *Citrullus vulgaris* Schr.; *Cucumis melo* L.; *Cucumis sativus* L.; *Lagenaria siceraria* Stand.; *Luffa cylindrica* Roem.; *Momordica charantis* L.; *Tricosanthes cucumeroids* Maxim.; *Tricosanthes kirilowii* Maxim. var *japonica* Kitam, and variants thereof.

4. A process as claimed in any one of the preceding claims wherein the substance having interferon inducing activity is extracted from the seed of the plant.

5. A process as claimed in any one of claims 1 to 4 wherein the extraction is effected by the use of a phenol/water mixture.

6. A process as claimed in claim 5 wherein the phenol concentration in the mixture is from 40 to 55%.

7. A process as claimed in any one of the preceding claims wherein the isolation is effected by fractionation of the supernatant by ultrafiltration to yield fractions containing the said IF inducer.

8. A process as claimed in claim 7 wherein the ultrafiltration is effected by the use of a membrane capable of retaining substances having a molecular weight of at least 30,000.

9. A process a claimed in claim 8 wherein the membrane is capable of retaining substances having a molecular weight of at least 50,000.

10. A polysaccharide IF inducer extracted from the tissue of a plant of the gourd family which is stable in the form of an amorphous whitish powder and which in substantially pure form possesses the following physico-chemical characteristics:

(1) Molecular weight: 30,000 to 200,000
(2) Melting point or decomposing point: Melting point indefinite. Carbonized at about 220°C.
(3) Ultraviolet absorption spectrum: Maximum absorption at about 258 nm; Colour reaction obtained by phenol/sulphuric acid method in maximum absorption at about 490 nm.
(4) Solubility in various solvents: Readily soluble in water and aqueous solutions of an alkali; Soluble in saturated aqueous solutions of phenol and dimethylsulfoxide; and substantially insoluble in methanol, ethanol, propanol, butanol, acetone, chloroform; diethyl ether, benzene and hexane.
(5) Reactions: Positive in Molish reaction, chromotropic acid reaction, tryptophan reaction and orcinol reaction, and negative in diphenylamine reaction.
(6) Nature: Acidic.
(7) Main chemical constituents: Phosphoric acid; rhamnose, arabinose, galactose and glucose moieties; xylose and/or mannose moieties; galacturonic acid and/or glucuronic acid moieties; and amino compounds.

11. A substance as claimed in claim 10 in substantially pure form.

**Revendications**

1. Procédé de préparation d'un inducteur d'interféron (IF) hydrosoluble, caractérisé en ce que l'inducteur de IF est extrait avec de l'eau ou un mélange phénol/eau du tissu d'une plante de la famille des cucurbitacées ou d'un variant de cette famille contenant l'inducteur de IF, de manière à former un produit surnageant et â isoler l'inducteur de IF du produit surnageant ainsi obtenu.

2. Procédé selon la revendication 1, caractérisé en ce que la plante est choisie parmi les genres Benincasa, Cucurbita, Citrullus, Cucumis, Lagenaria, Luffa, Momordica, Tricosanthes et leurs variants.

3. Procédé selon la revendication 2, caractérisé en ce que la plante est choisie parmi Benincasa hispida Cogn. Cucurbita moschata Dec., Cucurbita pepo L., Cucurbita maxima Dec., Citrullus vulgaris Schr., Cucumis melo L. Cucumis sativus L., Lagenaria siceraria Stand., Luffa cylindrica Roem., Momordica charantis L., Tricosanthes cucumeroids Maxim. Tricosanthes kirilowii Maxim. var. Japonica Kitam et leurs variants.

4. Procédé selon l'une quelconque des revendications précédentes, caractérisé en ce que la substance ayant une activité d'inducteur d'interféron est extraite de la graine de la plante.

5. Procédé selon l'une quelconque des revendications 1 à 4, caractérisé en ce que l'extraction est effectuée en utilisant un mélange phénol/eau.

6. Procédé selon la revendication 5, caractérisé en ce que la concentration en phénol dans le mélange est comprise entre 40 et 55%.

7. Procédé selon l'une quelconque des revendications précédentes, caractérisé en ce que l'isolement est effectué par fractionnement du produit surnageant par ultrafiltration pour obtenir des fractions contenant l'inducteur de IF.

8. Procédé selon la revendication 7, charactérisé en ce que l'ultrafiltration est effectuée en utilisant une membrane capable de retenir des substances ayant une masse moléculaire d'au moins 30 000.

9. Procédé selon la revendication 8, caractérisé en ce que va membrane est capable de retenir des substances ayant une masse moléculaire d'au moins 50 000.

10. Inducteur de IF de polysaccharide, extrait du tissu d'une plante de la famille des cucurbitacées, stable sous forme d'une poudre amorphe blanchâtre et qui sous une forme pratiquement pure possède les propriétés physicochimiques suivantes:

(1) Masse moléculaire; de 30.000 à 200.000.
(2) Point de fusion ou point de décomposition: point de fusion indéfini. Carbonisation vers 220°C.
(3) spectre d'absorption ultraviolette: absorption maximale vers 258 nm; réaction colorée obtenue selon la méthode au phénol/acide sulfurique à une absorption maximale d'environ 490 nm.

# 0 030 812

(4) Solubilité dans différents solvants: facilement soluble dans l'eau et des solutions aqueuses d'un alcalin; soluble dans des solutions aqueuses saturées de phénol et de diméthylsulfoxyde; et pratiquement insoluble dans le méthanol, l'éthanol, le propanol, le butanol, l'acétone, le chloroforme; l'éther diéthylique, le benzène et l'hexane.

(5) Réactions: positive dans la réaction de Molish, la réaction à l'acide chromotropique, la réaction au tryptophane, et la réaction à l'orcinol, et négative dans la réaction à la diphénylamine.

(6) Nature: acide.

(7) Principaux constituants chimiques: acide phosphorique; unités rhamnose, arabinose, galactose et glucose; unités xylose et/ou mannose; unités acide galacturonique et/ou acide glucuronique; et composés aminés.

11. Substance selon la revendication 10, sous une forme pratiquement pure.

## Patentansprüche

1. Verfahren zur Herstellung eines wasserlöslichen Interferon-Induktors, dadurch gekennzeichnet, daß der Interferon-Induktor mit Wasser oder einer Phenol/Wasser-Mischung aus dem Gewebe einer Pflanze der Kürbisfamilie (*Cucurbitaceae*) oder einer Variante derselben, die den Interferon-Induktor enthält, extrahiert wird, wobei ein Obenstehendes gebildet wird, aus dem der Interferon-Induktor isoliert wird.

2. Verfahren nach Anspruch 1, dadurch gekennzeichnet, daß die Pflanze aus den Gattungen *Benincasa, Cucurbita, Citrullus, Cucumis, Lagenaria, Luffa, Momordica, Tricosanthes* oder Varianten derselben ausgewählt wird.

3. Verfahren nach Anspruch 2, dadurch gekennzeichnet, daß die Pflanze aus *Benincasa hispida* Cogn.; *Cucurbita moschata* Dec.; *Cucurbita pepo* L.; *Cucurbita maxima* Dec.; *Citrullus vulgaris* Schr.; *Cucumis melo* L.; *Cucumis sativus* l.; *Lagenaria siceraria* Stand.; *Luffa cylindrica* Roem.; *Momordica charantis* L.; *Tricosanthes cucumeroides* Maxim.; *Tricosanthes kirilowii* Maxim. var *japonica* Kitam und Varianten derselben ausgewählt wird.

4. Verfahren nach einem der vorhergehenden Ansprüche, dadurch gekennzeichnet, daß die Substanz mit Interferon induzierender Aktivität aus dem Samen der Pflanze extrahiert wird.

5. Verfahren nach einem der Ansprüche 1 bis 4, dadurch gekennzeichnet, daß die Extraktion unter Einsatz einer Phenol/Wasser-Mischung vorgenommen wird.

6. Verfahren nach Anspruch 5, dadurch gekennzeichnet, daß die Phenolkonzentration in der Mischung zwischen 40 und 55% liegt.

7. Verfahren nach einem der vorhergehenden Ansprüche, dadurch gekennziechnet, daß die Isolierung durch Fraktionierung des Obenstehenden im Wege der Ultrafiltration vorgenommen wird, um den Interferon-Induktor enthaltende Fraktionen zu erhalten.

8. Verfahren nach Anspruch 7, dadurch gekennzeichnet, daß die Ultrafiltration unter Verwendung einer Membran durchgeführt wird, von der Substanzen mit einem Molekulargewicht von wenigstens 30 000 zurückhaltbar sind.

9. Verfahren nach Anspruch 8, dadurch gekennzeichnet, daß von der Membran Substanzen mit einem Molekulargewicht von wenigstens 50 000 zurückhaltbar sind.

10. Ein aus dem Gewebe einer Pflanze der Kürbisfamilie extrahierter Polysaccharid-Interferon-Induktor, der in der Form eines amorphen weißlichen Pulvers stabil ist und in im wesentlichen reiner Form die folgenden physikochemischen Eigenschaften aufweist:

(1) Molekulargewicht: 30 000 bis 200 000

(2) Schmelz- oder Zersetzungspunkt: Schmelzpunkt unbestimmt. Verkohlt bei etwa 220°C.

(3) Ultraviolett-Absorptionsspektrum: Maximale Absorption bei etwa 258 mm; Farbreaktion erhalten bei der Phenol/Schwefelsäure-Methode in der maximalen Absorption bei etwa 490 mm.

(4) Löslichkeit in verschiedenen Lösungsmitteln: Schnell löslich in Wasser und wässrigen Alkolilösungen; löslich in gesättigten wässrigen Lösungen von Phenol und Dimethylsulfoxid; im wesentlichen unlöslich in Methanol, Äthanol, Propanol, Butanol, Azeton, Chloroform, Diäthyläther, Benzol und Hexan.

(5) Reaktionen: Positiv in der Molish-Reaktion, chromotopischen Säure-Reaktion, Tryptophan-Reaktion und Orcinal-Reaktion und negative in der Diphenylamin-Reaktion.

(6) Natur: Säuerlich

(7) Chemische Hauptbestandteil: Phosporsäure; Rhamnose-, Arabinos-, Galaktose- und Glukoseteile; Xylose- und/oder Mannoseteile; Galakturonsäure- und/oder Glukuronsäureteile und Aminoverbindungen.

11. Substanz nach Anspruch 10 in im wesentlichen reiner Form.